# EUROPEAN PATENT SPECIFICATION

(11) **EP 2 082 735 B1**
(45) Date of publication and mention of the grant of the patent: **29.09.2010**
(21) Application number: 08150576.0
(22) Date of filing: 23.01.2008
(51) Int. Cl.: A61K 9/16, A61K 31/496

(54) **Amorphous Aripiprazole and Process for the Preparation thereof**
Amorphes Aripiprazol und Verfahren zu seiner Herstellung
Aripiprazole amorphe et son procédé de préparation

(43) Date of publication of application: 29.07.2009
(73) Proprietor: Helm AG, 20097 Hamburg (DE)
(72) Inventor: Dreyer, Katja, D - 22337 Hamburg (DE); Löffler, Uwe, 25436, Tornesch (DE); Glänzer, Klaus, 22337, Hamburg (DE)
(74) Representative: UEXKÜLL & STOLBERG

(56) References cited:
- EP-A- 1 880 714
- WO-A-03/026659

## Description

### FIELD OF INVENTION

The present invention relates to oral formulations containing morphologically stabilized amorphous aripiprazole which has enhanced dissolution abilities. Another aspect of the invention relates to pharmaceutical dosage forms containing stabilized amorphous aripiprazole which can be used for instance for the treatment of schizophrenia and psychotic disorders.

### BACKGROUND OF THE INVENTION

Aripiprazole, 7-{4-[4-(2,3-dichlorophenyl)-1-piperazinyl]-butoxy}-3,4-dihydro carbostyril or 7-{4-[4-(2,3-dichlorophenyl)-1-piperazinyl]-butoxy}-3,4-dihydro-2(1H)-quinolinone, is an atypical antipsychotic agent useful for the treatment of schizophrenia. Schizophrenia is a common type of psychosis characterized by delusions, hallucinations and extensive withdrawal from others. The preparation of aripiprazole and other carbostyril derivatives is described in U.S. Pat. No. 4,734,416 and U.S. Pat. No. 5,006,528. These processes do not result in the formation of well defined, reproducible forms of aripiprazole.

Aripiprazole is known in several polymorphic forms. The Proceedings of the 4th Japanese-Korean Symposium on Separation Technology (Oct. 6-8, 1996) state that, aripiprazole anhydride crystals exist as anhydrous type-I crystals (conventional anhydride), anhydrous type-II crystals, and type III hydrate (conventional hydrate); the type-I crystals of aripiprazole anhydride (conventional anhydride) can be prepared by recrystallization from an ethanol solution of aripiprazole, or by heating aripiprazole hydrate at 80° C. The type-II crystals of aripiprazole anhydride can be prepared by heating conventional anhydride at 130° to 140°C for 15 hours. Type III crystals (conventional hydrate) were recrystallized from alcoholic solvent containing up to 20% (v/v) of water.

The hygroscopicity of the conventional anhydride crystals makes them difficult to handle since costly and burdensome measures must be taken in order to ensure that they are not exposed to moisture during preparation and formulation. When exposed to moisture, the conventional anhydrous forms of aripiprazole absorb water and convert to hydrous forms such as the monohydrate. The hydrous forms of aripiprazole have the disadvantage of being less soluble than the anhydrous forms. Moreover, variation in the amount of hydrous versus anhydrous aripiprazole from batch to batch makes it difficult to meet the specifications set by drug regulatory authorities. Furthermore, the solubility of aripiprazole is pH-dependent which influence the dissolution rate.

Numerous attempts have been made to overcome these disadvantages.

To ensure appropriate dissolution of poorly soluble drugs the drugs are commonly used in particle sizes smaller 50 µm. The small particle sizes are usually obtained by energy involved processes like extensive milling, micronization or grinding.

EP 1 330 249 and EP 1 419 776 disclose polymorphic modifications of aripiprazole which are said to have reduced hygroscopicity, such as anhydride B. Anhydride B can be obtained by milling conventional hydrate to provide intermediary hydrate A (particle size less 50 µm), which is converted to aripiprazole form B by heating to 90-125°C for 3 to 50 hours. Anhydride B can be converted to a glassy state by heating to about 170 °C and cooling to room temperature. The glassy form of aripiprazole anhydride forms aripiprazole anhydride crystals of form G upon storing. In addition to hydrate A and anhydrides B and G aripiprazole anhydride crystals of forms C to F are described.

EP 1 606 262 discloses two crystalline forms of aripiprazole and four crystalline forms of aripiprazole hydrochloride which are said to be stable over the time and to be reproducible.

WO 2005/009990 discloses crystalline forms of aripiprazole, aripiprazole methanolate and aripiprazole ethylene dichloride solvate. These crystalline forms of aripiprazole are said to be non hygroscopic and to have no tendency to convert to other forms.

WO 2004/106322 describes the preparation of polymorphic forms II, II and IV of aripiprazole. Form II is described as having a melting point of 133 to 135 °C, Form III of 122 to 125 °C and Form IV of 146 to 149 °C.

WO 2005/058835 discloses anhydrous aripiprazole crystalline forms I, II, VI, VIII, X, XI, XII, XIV, XIX, XX and methods for preparing the same. These forms of aripiprazole are said to be non-hygroscopic and to maintain compound stability during storage. They can be prepared directly by slurrying without heating a preexisting hydrate crystal form.

EP 1 398 040 discloses a medicinal composition prepared by dissolving or dispersing a drug like aripiprazole and a pH-independent, water-insoluble polymer in a wax-like or glycerine ester or fatty acid like low-melting-point substance or dispersing a drug in a mixture of both.

None of the above forms of aripiprazole is completely satisfactory. For instance, during approval of aripiprazole tablets in Europe and the United States the 20- and 30-mg tablets had to be redesigned because they exhibited less than complete and slower dissolution than other aripiprazole tablet strengths at pH 1.2 (see www.fda.gov/cder/foi/nda/2002/21-436_Abilify_biopharmr_P2). In spite of this effort, the manufacturer had to recall several batches of tablets due to dissolution failure (FDA, Enforcement Report 25.08.2004 and 06.07.2005).

EP 1 880 714, which falls under Art. 54(3) EPC and is thus not relevant to the question of inventive step, relates to morphologically stabilized amorphous aripiprazole and processes for preparing the same.

As can be seen from the above, there is a strong need for aripiprazole having a stable morphology and showing defined and stable dissolution rates. Moreover, due to the poor performance of the marketed products, the development of a stable and reproducibly bioavailable formulation of aripiprazole tablets is highly desired. None of the above mentioned forms or formulations have demonstrated sufficient bioavailability.

It is an object of the invention to provide aripiprazole in morphologically stable form with good solubility in aqueous systems which can be easily prepared.

### SUMMARY OF THE INVENTION

The present invention relates to oral formulations containing morphologically stable amorphous aripiprazole which has enhanced dissolution abilities.

The invention provides aripiprazole or a pharmaceutically acceptable salt thereof in stabilized amorphous form. The preparation of the amorphous aripiprazole of the present invention does not require grinding, the use of water-free conditions or the use of special polymorphic forms as starting materials. The dissolution rate of the stabilized amorphous aripiprazole of the present invention is not influenced by the formation of hydrates during storage and does therefore not require protection against moisture during storage.

Another aspect of the invention relates to pharmaceutical compositions comprising morphologically stabilized amorphous aripiprazole which can be used e.g. for the treatment of schizophrenia and psychotic disorders.

A further aspect of the invention relates to a process for preparing morphologically stabilized amorphous aripiprazole.

The above objects are achieved by pharmaceutical compositions comprising amorphous aripiprazole or a pharmaceutically acceptable salt thereof and at least a polymethacrylate as first stabilizing agent and a polyethylene glycol as second stabilizing agent.

### BRIEF DESCRIPTION OF THE FIGURES

Figure 1 shows the powder X-ray diffraction pattern of aripiprazole stabilized with polyethylene glycol (Macrogol 6000) and polyvinyl pyrrolidone (Kollidon VA 64) according to Example 1 (comparative). The observed peaks are due to matrix only (compare Figure 2).
Figure 2 shows the powder X-ray diffraction pattern of aripiprazole stabilized with polyethylene glycol (Macrogol 6000) and polyvinyl pyrrolidone (Kollidon VA 64) according to example 1 (comparative), the powder X-ray diffraction pattern of a mixture of polyethylene glycol (Macrogol 6000) and polyvinyl pyrrolidone (Kollidon VA 64) without aripiprazole, and the difference spectrum of the first and second spectrum.
Figure 3 shows the powder X-ray diffraction pattern of aripiprazole stabilized with polyethylene glycol (Macrogol 4000) and a pH-dependent water soluble polymethacrylate (Eudragit EPO) in the presence of tartaric acid according to Example 3. The observed peaks are due to the crystalline tartaric acid.
Figure 4 shows the dissolution profile of the tablets described in example 4.
Figure 5 shows the results of a biostudy with the tablets of example 4 (logarithmic profile of the mean).

### DETAILED DESCRIPTION OF THE INVENTION

The present invention for the first time provides amorphous aripiprazole or a pharmaceutically acceptable salt thereof which is morphologically stable as well as pharmaceutical composition comprising morphologically stable amorphous aripiprazole or a salt thereof. The pharmaceutical compositions of the present invention contain at least two stabilizing agents, i.e. agents which stabilize aripiprazole in its amorphous form and prevent crystallization thereof. In the following, these mixtures of amorphous aripiprazole with stabilizing agents will also be referred to as morphologically stabilized amorphous aripiprazole or stabilized amorphous aripiprazole.

The morphologically stabilized amorphous aripiprazole of the present invention may comprise minor amounts of crystalline aripiprazole. Preferably the amorphous aripiprazole of the invention comprises less than 20 % by weight of crystalline aripiprazole, more preferably less than 10 % by weight, even more preferably less than 5 % by weight, e.g. less than 2 % by weight, based on the total amount of aripiprazole. Most preferably the stabilized aripiprazole of the present invention comprises substantially no crystalline aripiprazole.

The stabilized aripiprazole of the present invention preferably comprises aripiprazole and stabilizing agents in a weight ratio of 1:20 to 5:1. According to one preferred embodiment of the present invention the compositions comprise 4.8 to 83.3 wt.-% of aripiprazole, based on the total weight of the composition. It is further preferred that the compositions comprise 3.8 to 66.7 wt.-% of amorphous aripiprazole, i.e. the amount of crystalline aripiprazole is within a range of 0 to 16.6 wt.-%, based on the total weight of the composition.

It was surprisingly found that the stabilized amorphous aripiprazole of the present invention is better soluble in aqueous systems than the known crystalline forms of aripiprazole. It can be stored for prolonged time periods without changing its morphological structure, i.e. without crystallization. It was particularly surprising to find that the storage stability is not impaired by the presence of minor amounts of crystalline aripiprazole which would have been expected to induce crystallization of the whole material. Even after storage the morphologically stabilized amorphous aripiprazole of the present invention shows no significant change in the dissolution abilities.

Surprisingly, a pharmaceutical formulation containing morphologically stabilized aripiprazole according to the invention shows advantageous biopharmaceutical properties. Preferably, an oral formulation containing stabilized amorphous aripiprazole according to the invention can be characterized by a bioavailability with an average Cₘₐₓ larger than 25 ng/ml of aripiprazole, an average tₘₐₓ below 4 hours and an average AUC_{∞} larger than 1500 ng·h/ml.

As a consequence of the observed bioavailability in healthy volunteers, the invention therefore provides a pharmaceutical composition comprising morphologically stabilized amorphous aripiprazole which can be used e.g. for the treatment of schizophrenia and psychotic disorders.

According to the present invention aripiprazole is stabilized in amorphous form by admixture with at least two stabilizing agents which are selected from the group consisting of embedding materials and/or solubilizers.

Preferred stabilizing agents are polyvinyl pyrrolidone, polymethacrylate, polyvinyl acetate phthalate, alkyl cellulose, hydroxyl alkyl cellulose, polyethylene glycol, polyethylene castor oil, polyethylene glycol sorbitan fatty acid, polyethylene polypropylene glycol, polyethylene oxide, polyoxyethylene alkyl ether, polyoxyethylene stearate, and derivatives of these substances, and mixtures thereof.

More preferred stabilizing agents are selected from polyvinyl pyrrolidone, polymethacrylate, polyethylene glycol, polyethylene castor oil, polyoxyethylene hydrogenated castor oil, polyethylene glycol sorbitan fatty acid, polyoxyethylene stearate, and mixtures thereof.

Particularly preferred stabilizing agents are selected from polyethylene glycol having an average molecular weight within the range of 190 to 10,000, polyethylene oxide sorbitan monooleate, polyoxyl 40 hydrogenated castor oil, polyoxyl 35 castor oil, polyethylene glycol-15-hydroxystearate, pH-dependent water-soluble polymethacrylates, such as dimethyl aminoethyl methacrylate copolymer, and vinyl pyrrolidone-vinyl acetate copolymer.

The most preferred stabilizing agents are selected from polyethylene glycol having an average molecular weight within the range of 190 to 10,000, pH-dependent water-soluble polymethacrylates, such as dimethyl aminoethyl methacrylate copolymer, and vinyl pyrrolidone-vinyl acetate copolymer.

The stabilizing agents can further be used in combination with an acidic compound, preferably an organic acid, such as tartaric acid or citric acid.

The term "pH-dependent water-soluble polymethacrylate" as used herein refers to a polymethacrylate wherein solubility in aqueous solution is dependent from the pH of said solution. Polymethacrylates which are soluble in aqueous solution up to pH 5.0 are preferred. Particularly preferred pH-dependent water-soluble polymethacrylates are methacrylate copolymers having tertiary amino groups, such as dimethyl aminoethyl methacrylate copolymer, e.g. Eudragit E 100 or Eudragit EPO.

The stabilised amorphous aripiprazole of the present invention comprises two or more stabilising agents. The use of 2 to 5 and in particular 2 to 3 stabilising agents is preferred.

Compositions according to the invention comprise at least two different stabilizing agents. The stabilizing agents are preferably selected from embedding materials and/or solubilizers. More preferably at least one stabilizing agent is selected from the group of embedding materials and at least one stabilizing agent is selected from the group of solubilizers.

According to the invention embedding materials are understood to be a kind of matrix or carrier material. Preferable embedding materials are physiologically acceptable polymers or copolymers based on vinyl compounds. The polyvinyl compounds may optionally be functionalized. They are preferably substituted by pyrrolidone, alkyl, ether, phenyl, amino, alkyl amino, dialkyl amino, ammonium, carboxy acid ester, carboxy acid, carboxy acid amide and/or acetate groups. Additionally, embedding materials can be selected from alkyl cellulose or hydroxyl alkyl cellulose.

According to the invention solubilizers are understood to be surfactants, or solubilizing, solubility enhancing agents or cosolvents for aripiprazole or a salt thereof. Preferred solubilizers are physiologically acceptable polymers or copolymers of ethylene glycol. The polyethylene compounds may optionally be functionalized. They are preferably substituted by substituents selected from fatty acids, fatty acid esters, fatty acid amides, castor oils, ethers, higher alcohols, alkyl, phenyl, amino, alkyl amino, dialkyl amino, or polyalcohol groups.

The first stabilizing agent is selected from polymethacrylates and mixtures thereof.

More preferably the first stabilizing agent is selected from amino alkyl methacrylate copolymer, methacrylic acid ester copolymer, ammonium alkyl methacrylate copolymer, and mixtures thereof.

Particularly preferably the first stabilizing agent is selected from pH-dependent water-soluble polymethacrylates, such as dimethyl aminoethyl methacrylate copolymers, and mixtures thereof.

According to a particularly preferred embodiment, the first stabilizing agent is a p H-dependent water-soluble polymethacrylate, such as a dimethyl aminoethyl methacrylate copolymer, in combination with an acidic compound, preferably an organic acid, such as tartaric acid or citric acid. Preferably the pH-dependent water-soluble polymethacrylate and the acidic compound are used in a weight ratio of 50:1 to 10:1, more preferably 25:1 to 15:1.

The second stabilizing agent is selected from polyethylene glycol and mixtures thereof. Preferably the second stabilizing agent is selected from solubilizers. More preferably, the stabilizing agent is selected from compounds which cause a lowering of the melting point of an embedding compound and additionally have the property of a solubilizer.

More preferably the second stabilizing agent is selected from polyethylene glycol having an average molecular weight within the range of 190 to 10,000 and mixtures thereof.

The stabilized amorphous aripiprazole of the present invention preferably comprises 15 to 95 wt.-%, more preferably 25 to 93 wt.-% and most preferably 45 to 91 wt.-% of one or more stabilizing agents, based on the total weight of aripiprazole and stabilizing agents.

In addition to the aripiprazole or a salt thereof and the stabilizing agents the compositions of the invention comprises one or more pharmaceutically acceptable excipients.

The pharmaceutical compositions of the present invention have preferably the form of oral dosage forms, such as granules, pellets, capsules, and in particular tablets, such as flash-melt tablets, oral dispersible tablets, sustained release tablets and immediate release tablets.

The pharmaceutical compositions of the present invention preferably comprise:
1 to 99.5 wit.-%, more preferably 5 to 75 wt.-% and most preferably 20 to 65 wt.-% of stabilized amorphous aripiprazole, and
0.05 to 99 wt.-%, more preferably 25 to 95 wt.-% and most preferably 35 to 80 wt.-% of further excipients.

The compositions preferably contain 1 to 200 mg, more preferably 1 to 100 mg and most preferably 1 to 30 mg of aripiprazole per unit dose.

The known pharmaceutical excipients can be used to prepare the pharmaceutical formulations of the invention, such as fillers, lubricants, disintegration aids, wetting agents, agents to improve the flow behavior, antioxidants, flavors, taste masking agents, pigments, dyes, lubricants and other additives, e.g. as disclosed in "Die Tablette", W. A. Ritschel and A. Bauer-Brandl, 2nd ed., ECV-Edition Cantor publishers, 2002.

Preferred fillers are celluloses and cellulose derivatives, such as microcrystalline cellulose, native cellulose, hydroxypropyl cellulose, hydroxypropyl methyl cellulose, methyl cellulose, sugars, such as lactose, fructose, saccharose, glucose, maltose, sugar alcohols, such as lactitol, mannitol, sorbitol, xylitol, inorganic fillers, such as calcium phosphates and calcium sulfates, and starches, such as corn starch, potato starch, wheat starch, dextrins, and pregelatinized starches. Mannitol is particularly preferred.

Preferred disintegration aids are crosslinked polyvinyl pyrrolidone, starch and modified starches. Crosslinked polyvinyl pyrrolidone is particularly preferred.

Preferred flavors are vanilla, cherry, lemon, and cacao. Preferred taste masking agents are ascorbic acid, citric acid, tartaric acid, apple acid and polymethacrylate.

Preferred lubricants are fatty acids, such as stearic acid, fumaric acid, and salts thereof.

Preferred sweetening agents are acesulfame potassium, cyclamate and its salts, aspartame, saccharin and its salts or mixtures thereof.

The tablets can be coated or uncoated. Sugar-coated tablets, gelatin-coated tablets, enteric coated tablets and film coated tablets are preferred as coated tablets. Furthermore, the tablets can be double tablets or multilayered tablets.

The pharmaceutical compositions of the present invention preferably have the form of immediate release tablets or orodispersible tablets.

Immediate release tablet preferably comprise:
1 to 99 wt.-%, preferably 25 to 70 wt.-% and more preferably 30 to 50 wt.-% of morphologically stabilized amorphous aripiprazole,
0 to 50 wt.-%, preferably 0.5 to 50 wt.-%, more preferably 2 to 25 wt.-% and most preferably 4 to 15 wt.-% disintegration aids,
0 to 90 wt.-%, preferably 0.5 to 90 wt.-%, more preferably 10 to 60 wt.-% and most preferably 25 to 50 wt.-% filler,
0 to 5 wt.-% flavor, and
0 to 10 wt.-%, preferably 0.1 to 10 wt.-%, more preferably 0.3 to 5 wt.-% and most preferably 0.5 to 3 wt.-% lubricant.

Orodispersible tablets preferably comprise:
1 to 99 wt.-%, preferably 3 to 50 wt.-% and more preferably 5 to 40 wt.-% morphologically stabilized amorphous aripiprazole,
0 to 10 wt.-%, preferably 0.3 to 6 wt.-% and more preferably 0.5 to 2 wt.-% taste masking agent,
0 to 90 wt.-%, preferably 0.5 to 90 wt.-%, more preferably 40 to 90 wt.-% and most preferably 70 to 85 wt.-% filler,
0 to 50 wt.-%, preferably 0.5 to 50 wt.-% disintegration aids,
0 to 5 wt.-%, preferably 0.1 to 5 wt.-%, more preferably 0.2 to 5 wt.-% and most preferably 0.3 to 3 wt.-% lubricant, and
0 to 5 wt.-% flavor.

The morphologically stabilized amorphous aripiprazole of the present invention can be prepared by mixing aripiprazole with at least a polymethacrylates as first stabilizing agent and a polyethylene glycol as second stabilizing agent.

Preferably the aripiprazole is dissolved in stabilizing agents at elevated temperature, preferably at a temperature within the range of 30 to 190 °C, and then the solution is optionally cooled.

According to a preferred embodiment of the invention, aripiprazole is dissolved in a mixture of at least two different stabilizing agents at elevated temperature and then the solution is optionally cooled. According to this embodiment of the invention the first stabilizing agent is selected from polymethacrylate and mixtures thereof.

Preferably the first stabilizing agent is a pH-dependent water-soluble polymethacrylate, such as a dimethyl aminoethyl methacrylate copolymer.

The second stabilizing agent is selected from polyethylene glycol and mixtures thereof.

Preferably the second stabilizing agent is selected from polyethylene glycol with an average molecular weight from 190 to 10000.

Most preferably a mixture of a pH-dependent water-soluble polymethacrylate, such as a dimethyl aminoethyl methacrylate copolymer, and polyethylene glycol with an average molecular weight from 190 to 10000, particularly about 2000 to 8000 is used.

The first stabilizing agent, the second stabilizing agent and aripiprazole are preferably used in a weight ratio of 10:10:1 to 0.5:0.5:1, more preferably 5:5:1 to 2:2:1.

The active ingredient and the stabilizing agents are preferably mixed at a temperature within the range of 40 to 190°C, more preferably 100 to 180°C, most preferably 140° to 180°C. It is particularly preferred that the active ingredient and the stabilizing agents are mixed and heated in an extruder, such as but not limited to a hot-melt-extruder, at a temperature within the range of 40 to 190°C, more preferably 100 to 180°C, most preferably 140° to 150°C, followed by extrusion of the mixture.

The first stabilizing agent and the second stabilizing agent are preferably used in a weight ratio of 10:1 to 1:10, more preferably 5:1 to 1:2, most preferably 1:1. Alternatively, the first stabilizing agent and the second stabilizing agent are used in a weight ratio of 4.9:1 to 4:1.

Aripiprazole is preferably used in an amount of 5 to 50 wt.-%, based on the total weight of aripiprazole and stabilizing agents.

According to a further preferred embodiment of the invention aripiprazole is first dissolved in an organic solvent at elevated temperature, followed by the addition of at least one stabilizing agent. Alternatively, the aripiprazole may be added to a solution of the stabilizing agents in an organic solvent. The compounds are mixed until a solution is obtained and then the organic solvent is removed.

Preferred organic solvents are ethanol, ethyl acetate, methanol, isopropyl alcohol, isopropyl acetate, dichloro methane, toluene, chloroform, preferred ethanol, acetone and mixtures thereof.

If an organic solvent is used, at least two different stabilizing agents are used as defined above.

According to the present invention it is particularly preferred to use a combination of polyethylene glycol with an average molecular weight from 190 to 10000, particularly about 2000 to 8000, and a pH-dependent water-soluble polymethacrylate, such as a dimethyl aminoethyl methacrylate copolymer, as stabilizing agents.

The first stabilizing agent, the second stabilizing agent and aripiprazole are preferably used in a weight ratio of 10:10:1 to 0.5:0.5:1, more preferably 4.5:4.5:1. The active ingredient, the organic solvent and the stabilizing agents are preferably mixed at a temperature within the range of 40 to 120°C, more preferably 50 to 90°C, most preferably 60° to 75°C.

The first stabilizing agent and the second stabilizing agent are preferably used in a weight ratio of 10:1 to 1:10, more preferably 5:1 to 1:2, most preferably 1:1. Alternatively, the first stabilizing agent and the second stabilizing agent are used in a weight ratio of 4.9:1 to 4:1.

Aripiprazole is preferably used in an amount of 5 to 50 wt.-%, based on the total weight of aripiprazole and stabilizing agents.

To prepare morphologically stabilized amorphous aripiprazole any known polymorphic form, salt or solvate of aripiprazole can be used, for example conventional anhydride, conventional hydrate, type III, form A, form B, form I, form II, form III, form IV, VI, VIII, X, XI, XII, XIV, XIX, XX. The preparation is not limited to anhydride polymorphic forms of aripiprazole. Aripiprazole of any particle size may be used. No previous grinding is necessary. Preferred salts of aripiprazole are the acid-addition salts, in particular the salts of hydrochloric acid, sulfuric acid, phosphoric acid, hydrobromic acid, oxalic acid, maleic acid, fumaric acid, malic acid, tartaric acid, citric acid, and benzoic acid.

The morphologically stabilized amorphous aripiprazole obtained by any of the above procedures can be used directly for the preparation of pharmaceutical compositions or may be first subjected to grinding, sieving, or milling.

Oral pharmaceutical formulations can be produced for instance by direct compression, dry-granulation, wet-granulation, preferably by melt extrusion, melt granulation or pelletization. Particularly, oral pharmaceutical formulations can be produced by direct compression of a dry mixture of the morphologically stabilized amorphous aripiprazole or pharmaceutically acceptable salt thereof and one or more pharmaceutically acceptable excipients, or by compression of a granulate manufactured from the morphologically stabilized amorphous aripiprazole or pharmaceutically acceptable salt thereof and one or more pharmaceutically acceptable excipients by wet-granulation.

Dissolution rates of the pharmaceutical compositions and formulations according to the invention can be determined according to USP (900 ml buffer pH 1.2, 37°C, 60 rpm). Preferably, the pharmaceutical compositions and formulations according to the invention exhibit a dissolution profile wherein under the above conditions within 5 minutes at least 95 wt.-%, particularly at least 98 wt.-%, most preferably at least 99.5 wt.-% of aripiprazole are dissolved.

### EXAMPLES

For the preparation of morphologically stabilized amorphous aripiprazole the conventional anhydride of aripiprazole prepared according to Example 1 of EP 0 367 141 A2 was used.

All X-ray diffraction measurements were performed in the transmission mode using the following conditions:
Cu- Kα₁- radiation (λ=1.54056Å), U = 40kV, I = 30mA.
Primary Monochromator (curved Ge(111)).
Position Sensitive Detector, sample movement Δω.
Slits: 1 mm, 8 mm diameter.
Angle region: 2θ = 3 to 35°, steps Δ2θ = 0,2° (0,02°), t = 18s / step

### Example 1 (comparative): Preparation of stabilized amorphous aripiprazole by mixing the active ingredient with two stabilizing agents (Macrogol 6000/Kollidon VA 64) in the absence of organic solvent

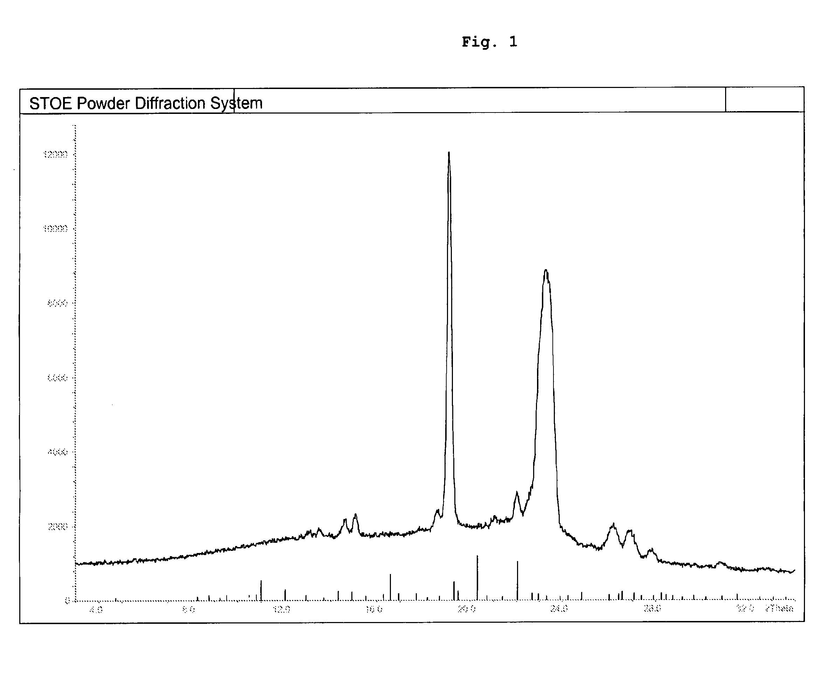

10 g of polyethylene glycol (Macrogol 6000) and 10 g of polyvinyl pyrrolidone (Kollidon VA 64) were heated in a glass vessel to T ≈ 150°C to 180°C. At that temperature 4.98 g aripiprazole (Example 1) were added with stirring to obtain a homogeneous solution. Stirring was continued for an additional 10 min., the mixture was poured into a mortar and allowed to cool down to room temperature. X-ray diffraction measurement of the product resulted in the spectrum shown in Fig. 1.

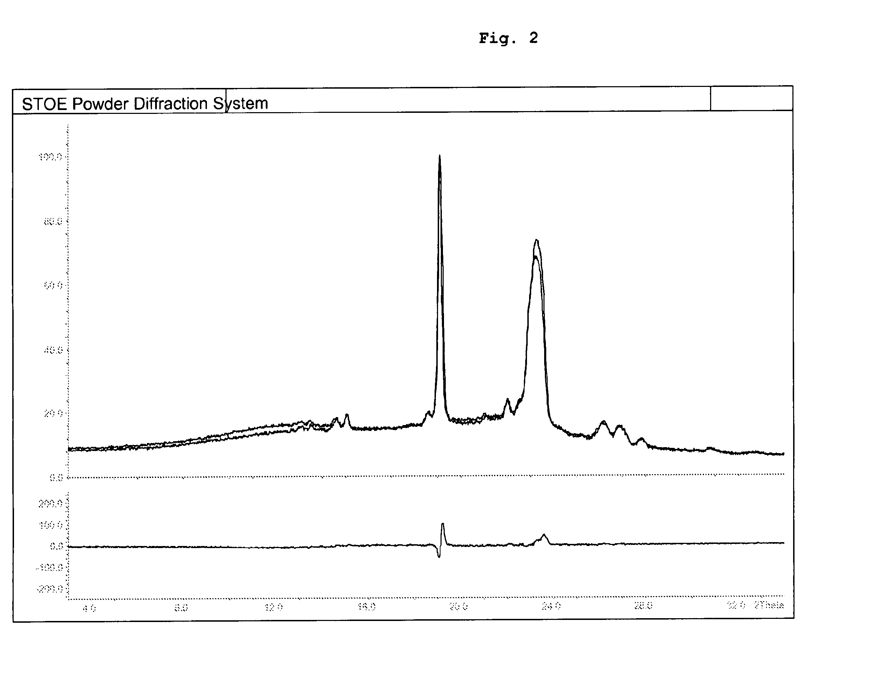

In the upper part of Fig. 2 the spectrum of Fig. 1 is overlaid with the spectrum of a mixture of Macrogol 6000 and Kollidon VA 64 without the active ingredient. In the lower part of Fig. 2 the difference of the two spectra is shown. It can be seen that the peaks of Fig. 1 must be caused by the stabilizing agents, i.e. the aripiprazole contained in the mixture is purely amorphous and does therefore not result in the formation of any additional peaks.

### Example 2 (comparative): Preparation of stabilized amorphous aripiprazole by mixing the active ingredient with two stabilizing agents (Macrogol 6000/Kollidon VA 64) in the absence of organic solvent

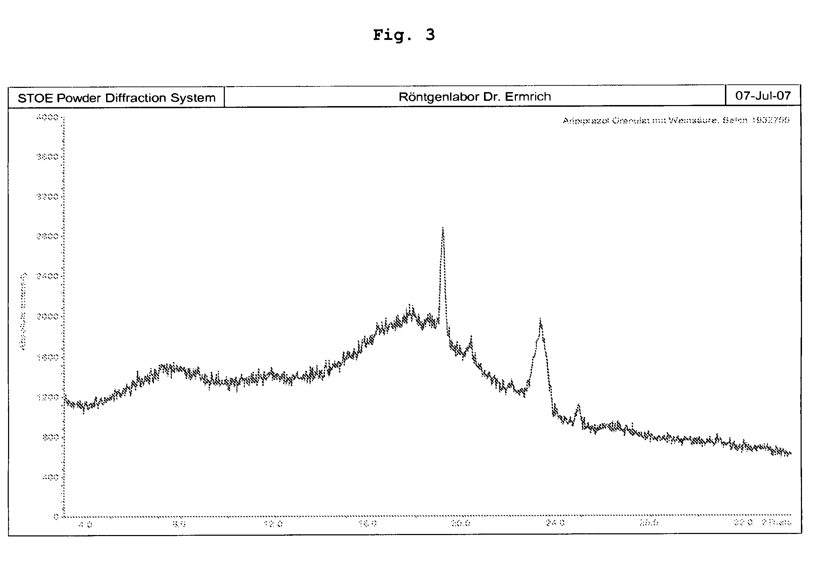

5.63 g of polyethylene glycol (Macrogol 6000) and 5.64 g of polyvinyl pyrrolidone (Kollidon VA 64) were heated in a glass vessel to T ≈ 150°C to 180°C. At that temperature 1.32 g aripiprazole were added with stirring to obtain a homogeneous solution. Stirring was continued for an additional 10 min., then the dispersion was poured into a mortar and allowed to cool down to room temperature. X-ray diffraction measurement of the product resulted in the spectrum shown in Fig. 3.

### Example 3: Preparation of stabilized amorphous aripiprazole by mixing the active ingredient with two stabilizing agents (Macrogol 4000/Eudragit EPO)

1.45 kg of polyethylene glycol (Macrogol 4000), 0.31 kg of Tartaric Acid, 6.79 kg of a pH-dependent water-soluble polymethacrylate (Eudragit EPO) and 1.45 kg of Aripiprazol were mixed and heated in a hot-melt-extruder (Leistritz ZSE-27 HP-40D) to T ≈ 140°C to 150°C. After the extrusion process the resulting extrudate was allowed to cool down to room temperature and milled.

### Example 4: Tablet containing stabilized amorphous aripiprazole in a dosage strength of 10 mg aripiprazole

Tablets of the following composition were prepared by direct compression of mixture of the substances specified in Table I on tabletting machine (Fette P 1200).

| **Immediate release tablet** | | |
|---|---|---|
| **Component** | **wt.-%** | **mg per tablet** |
| stabilized amorphous aripiprazole (Ex 3) | 34,48 | 68,97¹⁾ |
| Microcrystalline Cellulose | 15,0 | 30,00 |
| Mannitol | 43,52 | 87,03 |
| Crosslinked Polyvinylpyrollidone | 5,0 | 10,00 |
| Sweeteners | 0,9 | 1,80 |
| Aroma | 0,1 | 0,20 |
| Magnesium stearate | 1,0 | 2,0 |
| **Total** | **100** | **200,0** |

| | | |
|---|---|---|
| ¹⁾ = 10 mg aripiprazole | | |

### Example 5: Dissolution profile of the tablets of example 4 (Batch-No. 74621)

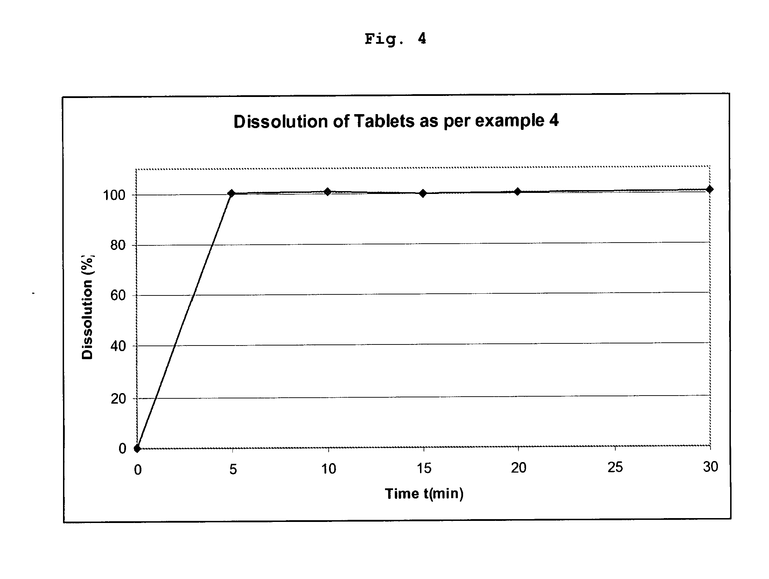

The dissolution rate of the pharmaceutical composition of example 4 was determined according to USP (900 ml buffer pH 1.2, 37°C, 60 rpm) using an amount of stabilized aripiprazole which corresponds to 10 mg of pure aripiprazole. The dissolution profile is shown in Fig. 4.

### Example 6: Results of Biostudy with the tablets of Example 4 (Batch-No. 74621; Assay 96.4%)

| **PARAMETER** | **Tablets of Example 4** | |
|---|---|---|
| | **MEAN** | **C.V.** |
| Cₘₐₓ (ng/mL) | 46,26 | 46,5 |
| Tₘₐₓ (hours) | 3,17 | 80,5 |
| AUC_{T} (ng·h/mL) | 1372,86 | 25,2 |
| AUC_{∞} (ng·h/mL) | 2477,40 | 29,3 |
| Kₑₗ (hours⁻¹) | 0,0121 | 35,1 |
| T_{½el} (hours) | 65,05 | 41,1 |

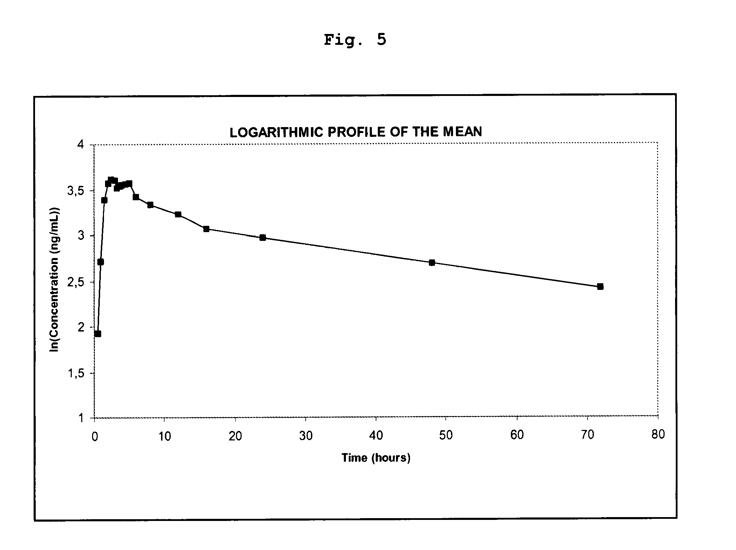

The logarithmic profile of the mean is shown in Fig. 5.

### Example 7: Tablets containing stabilized amorphous aripiprazole in different dosage strengths of aripiprazole.

| | Dosage strength: 5 mg aripiprazole | Dosage strength: 15 mg aripiprazole | Dosage strength: 20 mg aripiprazole | Dosage strength: 30 mg aripiprazole |
|---|---|---|---|---|
| **Component** | **mg per tablet** | **mg per tablet** | **mg per tablet** | **mg per tablet** |
| stabilized amorphous aripiprazole of example 3 | 34,49 | 103,45 | 137,94 | 206,91 |
| Microcrystalline Cellulose | 15,00 | 45,00 | 60,00 | 90,00 |
| Mannitol | 43,51 | 130,55 | 174,06 | 261,09 |
| Crosslinked Polyvinylpyrrolidone | 5,00 | 15,00 | 20,00 | 30,00 |
| Sweeteners | 0,90 | 2,70 | 3,60 | 5,40 |
| Aroma | 0,10 | 0,30 | 0,40 | 0,60 |
| Magnesium stearate | 1,0 | 3,0 | 4,0 | 6,0 |
| **Total** | **100,0** | **300,0** | **400,0** | **600,0** |

## Claims

1. A composition comprising amorphous aripiprazole and at least
as first stabilizing agent a polymethacrylate, and
as second stabilizing agent a polyethylene glycol.

2. The composition of claim 1, wherein the polymethacrylate is a pH-dependent water-soluble polymethacrylate.

3. The composition of claim 2, wherein the polymethacrylate is a dimethyl aminoethyl methacrylate copolymer.

4. The composition of any one of claims 1 to 3, wherein the polyethylene glycol is a polyethylene glycol having an average molecular weight within the range of 190 to 10,000.

5. The composition of any one of claims 1 to 4, comprising the first stabilizing agent and the second stabilizing agent in a weight ratio of 5:1 to 1:5.

6. The composition of any one of claims 1 to 5, comprising 4.8 to 83.3 wt.-% of aripiprazole.

7. The composition of any one of claims 1 to 6, comprising 3.8 to 66.7 wt.-% of amorphous aripiprazole.

8. A process for preparing morphologically stabilized amorphous aripiprazole comprising the mixing of aripiprazole with a mixture of at least a polymethacrylate as first stabilizing agent and a polyethylene glycol as second stabilizing agent.

9. The process of claim 8, wherein the aripiprazole is dissolved in the mixture of at least a polymethacrylate as first stabilizing agent and a polyethylene glycol as second stabilizing agent.

10. The process of claim 8 or 9, wherein the aripiprazole and stabilizing agents are used in a weight ratio of 1:20 to 5:1.

11. The process of any one of claims 8 to 10, wherein the first stabilizing agent and the second stabilizing agent are used in a weight ratio of 5:1 to 1:5.

12. The process of any one of claims 8 to 11, wherein aripiprazole, first and second stabilizing agent are used in a weight ratio of 1:10:10 to 1:0.5:0.5.

13. The process of any one of claims 8 to 12, wherein the mixture is heated at a temperature of 40 to 190 °C.

14. The process of claim 13, wherein the active ingredient and the stabilizing agents are mixed and heated in an extruder, followed by extrusion of the mixture.

15. The composition of claim 1, comprising morphologically stabilized amorphous aripiprazole obtainable according to a process of any one of claims 8 to 14.

16. Use of at least a polymethacrylate as first stabilizing agent and a polyethylene glycol as second stabilizing agent for converting crystalline aripiprazole to morphologically stabilized amorphous aripiprazole.

17. A pharmaceutical composition comprising the composition of any one of claims 1 to 7 and 15, further comprising at least one pharmaceutically acceptable excipient.

18. The pharmaceutical composition of claim 17, comprising 1 to 99.5 wt.-% of morphologically stabilized amorphous aripiprazole, containing 0.1 to 95 wt.-% of stabilizing agent(s).

19. The pharmaceutical composition of claim 17 or 18, comprising:
1 to 99.5 wt.-% morphologically stabilized amorphous aripiprazole, and
0.5 to 99 wt.-% further excipients.

20. The pharmaceutical composition of any one of claims 17 to 19, comprising:
1 to 99 wt.-% of morphologically stabilized amorphous aripiprazole,
0.5 to 50 wt.-% disintegration aids, and
0.5 to 90 wt.-% filler.

21. The pharmaceutical composition of claim 20, further comprising:
0.1 to 10 wt.-% lubricant, and
0 to 5 wt.-% flavor.

22. The pharmaceutical composition of any one of claims 17 to 21 having a bioavailability with an average Cₘₐₓ larger than 25 ng/ml of aripiprazole, an average tₘₐₓ below 4 hours and an average AUC_{∞} larger than 1500 ng·h/ml.

23. Use of the composition of any of claims 1 to 7, 15 and 17 to 22 in the manufacture of an oral pharmaceutical formulation selected from flash-melt tablets, oral dispersible tablets, sustained release tablets and immediate release tablets.

## Patentansprüche

1. Zusammensetzung, die amorphes Aripiprazol und mindestens ein Polymethacrylat als erstes Stabilisierungsmittel und ein Polyethylenglykol als zweites Stabilisierungsmittel enthält.

2. Zusammensetzung nach Anspruch 1, bei der das Polymethacrylat ein pH-abhängiges wasserlösliches Polymethacrylat ist.

3. Zusammensetzung nach Anspruch 2, bei der das Polymethacrylat ein Dimethylaminoethylmethacrylat-Copolymer ist.

4. Zusammensetzung nach einem der Ansprüche 1 bis 3, bei der das Polyethylenglykol ein Polyethylenglykol mit einem durchschnittlichen Molekulargewicht im Bereich von 190 bis 10 000 ist.

5. Zusammensetzung nach einem der Ansprüche 1 bis 4, die das erste Stabilisierungsmittel und das zweite Stabilisierungsmittel in einem Gewichtsverhältnis von 5:1 bis 1:5 enthält.

6. Zusammensetzung nach einem der Ansprüche 1 bis 5, die 4,8 bis 83,3 Gew.% Aripiprazol enthält.

7. Zusammensetzung nach einem der Ansprüche 1 bis 6, die 3,8 bis 66,7 Gew.% amorphes Aripiprazol enthält.

8. Verfahren zur Herstellung von morphologisch stabilisiertem amorphem Aripiprazol, bei dem man das Aripiprazol mit einer Mischung aus dem mindestens einen Polymethacrylat als erstes Stabilisierungsmittel und einem Polyethylenglykol als zweites Stabilisierungsmittel mischt.

9. Verfahren nach Anspruch 8, bei dem man das Aripiprazol in der Mischung aus mindestens einem Polymethacrylat als erstes Stabilisierungsmittel und einem Polyethylenglykol als zweites Stabilisierungsmittel löst.

10. Verfahren nach Anspruch 8 oder 9, bei dem man das Aripiprazol und die Stabilisierungsmittel in einem Gewichtsverhältnis von 1:20 bis 5:1 verwendet.

11. Verfahren nach einem der Ansprüche 8 bis 10, bei dem man das erste Stabilisierungsmittel und das zweite Stabilisierungsmittel in einem Gewichtsverhältnis von 5:1 bis 1:5 verwendet.

12. Verfahren nach einem der Ansprüche 8 bis 11, bei dem man Aripiprazol, das erste und das zweite Stabilisierungsmittel in einem Gewichtsverhältnis von 1:10:10 bis 1:0,5:0,5 verwendet.

13. Verfahren nach einem der Ansprüche 8 bis 12, bei dem man die Mischung auf eine Temperatur von 40 bis 190°C erwärmt.

14. Verfahren nach Anspruch 13, bei dem man den Wirkstoff und die Stabilisierungsmittel in einem Extruder mischt und erwärmt und anschließend die Mischung extrudiert.

15. Zusammensetzung nach Anspruch 1, die morphologisch stabilisiertes amorphes Aripiprazol enthält, das gemäß einem Verfahren nach einem der Ansprüche 8 bis 14 erhältlich ist.

16. Verwendung von mindestens einem Polymethacrylat als erstes Stabilisierungsmittel und einem Polyethylenglykol als zweites Stabilisierungsmittel zur Umwandlung von kristallinem Aripiprazol in morphologisch stabilisiertes amorphes Aripiprazol.

17. Pharmazeutische Zusammensetzung, die die Zusammensetzung nach einem der Ansprüche 1 bis 7 und 15 enthält und ferner mindestens einen pharmazeutisch annehmbaren Hilfsstoff enthält.

18. Pharmazeutische Zusammensetzung nach Anspruch 17, die 1 bis 99,5 Gew.% morphologisch stabilisiertes amorphes Aripiprazol enthält, das 0,1 bis 95 Gew.% Stabilisierungsmittel enthält.

19. Pharmazeutische Zusammensetzung nach Anspruch 17 oder 18, die
1 bis 99,5 Gew.% morphologisch stabilisiertes amorphes Aripiprazol und
0,5 bis 99 Gew.% weitere Hilfsstoffe enthält.

20. Pharmazeutische Zusammensetzung nach einem der Ansprüche 17 bis 19, die
1 bis 99 Gew.% morphologisch stabilisiertes amorphes Aripiprazol,
0,5 bis 50 Gew.% Sprengmittel und
0,5 bis 90 Gew.% Füllstoffe enthält.

21. Pharmazeutische Zusammensetzung nach Anspruch 20, die ferner 0,1 bis 10 Gew.% Schmiermittel und 0 bis 5 Gew.%. Aroma enthält.

22. Pharmazeutische Zusammensetzung nach einem der Ansprüche 17 bis 21, die eine Bioverfügbarkeit mit einer durchschnittlichen Cₘₐₓ von mehr als 25 ng/ml Aripiprazol, einer durchschnittlichen tₘₐₓ unter 4 Stunden und einer durchschnittlichen AUC größer als, 1500 ng·h/ml aufweist.

23. Verwendung der Zusammensetzung nach einem der Ansprüche 1 bis 7, 15 und 17 bis 22 zur Herstellung einer oralen pharmazeutischen Formulierung ausgewählt aus Flash Melt-Tabletten (rasch zerfallenden Tabletten), oral dispergierbaren Tabletten, Tabletten mit verzögerter Freisetzung sowie Tabletten mit sofortiger Freisetzung.

## Revendications

1. Composition comprenant un aripiprazole amorphe et au moins
un polyméthacrylate comme premier agent de stabilisation, et
un polyéthylène glycol comme second agent de stabilisation.

2. Composition selon la revendication 1, dans laquelle le polyméthacrylate est un polyméthacrylate hydrosoluble pH dépendant.

3. Composition selon la revendication 2, dans laquelle le polyméthacrylate est un copolymère de diméthyl aminoéthyl méthacrylate.

4. Composition selon l'une quelconque des revendications 1 à 3, dans laquelle le polyéthylène glycol est un polyéthylène glycol ayant une masse moléculaire moyenne dans la plage de 190 à 10 000.

5. Composition selon l'une quelconque des revendications 1 à 4, comprenant le premier agent de stabilisation et le second agent de stabilisation dans un rapport en poids de 5:1 à 1:5.

6. Composition selon l'une quelconque des revendications 1 à 5, comprenant de 4,8 à 83,3 % en poids d'aripiprazole.

7. Composition selon l'une quelconque des revendications 1 à 6, comprenant de 3,8 à 66,7 % en poids d'aripiprazole amorphe.

8. Procédé de préparation d'aripiprazole amorphe stabilisé morphologiquement comprenant le mélange de l'aripiprazole avec un mélange d'au moins un polyméthacrylate comme premier agent de stabilisation et d'un polyéthylène glycol comme second agent de stabilisation.

9. Procédé selon la revendication 8, dans lequel l'aripiprazole est dissous dans le mélange d'au moins un polyméthacrylate comme premier agent de stabilisation et d'un polyéthylène glycol comme second agent de stabilisation.

10. Procédé selon la revendication 8 ou 9, dans lequel l'aripiprazole et les agents de stabilisation sont utilisés dans un rapport en poids de 1:20 à 5:1.

11. Procédé selon l'une quelconque des revendications 8 à 10, dans lequel le premier agent de stabilisation et le second agent de stabilisation sont utilisés dans un rapport en poids de 5:1 à 1:5.

12. Procédé selon l'une quelconque des revendications 8 à 11, dans lequel l'aripiprazole, et les premier et second agents de stabilisation sont utilisés dans un rapport en poids de 1:10:10 à 1:0,5:0,5.

13. Procédé selon l'une quelconque des revendications 8 à 12, dans lequel le mélange est chauffé à une température de 40 à 190 °C.

14. Procédé selon la revendication 13, dans lequel l'ingrédient actif et les agents de stabilisation sont mélangés et chauffés dans une extrudeuse, avant une extrusion du mélange.

15. Composition selon la revendication 1, comprenant un aripiprazole amorphe stabilisé morphologiquement pouvant être obtenu par un procédé selon l'une quelconque des revendications 8 à 14.

16. Utilisation d'au moins un polyméthacrylate comme premier agent de stabilisation et d'un polyéthylène glycol comme second agent de stabilisation pour convertir l'aripiprazole cristallin en aripiprazole amorphe stabilisé morphologiquement.

17. Composition pharmaceutique comprenant la composition selon l'une quelconque des revendications 1 à 7 et 15, comprenant en outre au moins un excipient pharmaceutiquement acceptable.

18. Composition pharmaceutique selon la revendication 17, comprenant de 1 à 99,5 % en poids d'aripiprazole amorphe stabilisé morphologiquement, contenant 0,1 à 95 % en poids d'agent(s) de stabilisation.

19. Composition pharmaceutique selon la revendication 17 ou 18, comprenant :
de 1 à 99,5 % en poids d'aripiprazole amorphe stabilisé morphologiquement, et
de 0,5 à 99 % en poids d'autres excipients.

20. Composition pharmaceutique selon l'une quelconque des revendications 17 à 19, comprenant :
de 1 à 99 % en poids d'aripiprazole amorphe stabilisé morphologiquement,
de 0,5 à 50 % en poids d'agents de désagrégation, et
de 0,5 à 90 % en poids de charge.

21. Composition pharmaceutique selon la revendication 20, comprenant en outre :
de 0,1 à 10 % en poids de lubrifiant, et
de 0 à 5 % en poids d'arôme.

22. Composition pharmaceutique selon l'une quelconque des revendications 17 à 21 ayant une biodisponibilité avec une Cₘₐₓ moyenne supérieure à 25 ng/ml d'aripiprazole, une tₘₐₓ moyenne inférieure à 4 heures et une SSC_{∞} moyenne supérieure à 1 500 ng.h/ml.

23. Utilisation de la composition selon l'une quelconque des revendications 1 à 7, 15 et 17 à 22 dans la fabrication d'une formulation pharmaceutique orale choisie parmi les comprimés à dissolution rapide, les comprimés orodispersibles, les comprimés à libération prolongée et les comprimés à libération immédiate.
